# EUROPEAN PATENT APPLICATION

(11) **EP 4 755 205 A1**
(43) Date of publication of application: **10.06.2026**
(21) Application number: 24217250.0
(22) Date of filing: 03.12.2024
(51) Int. Cl.: A24B 13/00, A24B 15/30, A24F 23/00, A61K 9/00, B65B 29/00

(54) **SMOKELESS ARTICLE**

(71) Applicant: Imperial Tobacco Limited, Bristol BS3 2LL (GB)
(72) Inventor: DE DOMINICIS, Mattia, Bristol, BS3 2LL (GB); JONES, Jill, Bristol, BS3 2LL (GB); ELLIS, Paul, Bristol, BS3 2LL (GB); OSBORNE, Victoria Lee, Bristol, BS3 2LL (GB)
(74) Representative: Elkington and Fife LLP

(57) **Abstract**

A smokeless article for oral delivery of a nicotinic component. The smokeless article comprises a water-permeable pouch having two or more compartments including a first compartment and a second compartment each containing a content, each compartment, or each content, comprising at least one of a nicotinic component and a flavouring. The format of the content contained in the first compartment is different to the format contained in the second component. Also disclosed is a method of manufacturing a smokeless article, a kit comprising a plurality of smokeless articles, and a use of a smokeless article.

## Description

### FIELD

The present disclosure relates to a smokeless article. In particular, the disclosure relates to a smokeless article for oral delivery of a nicotinic component. This disclosure also relates to methods of manufacturing the smokeless articles, kits comprising the smokeless articles and use of the smokeless articles.

### BACKGROUND

Smokeless articles are placed in the mouth where saliva extracts the soluble element from the content contained within. Typically, the smokeless article is placed in the oral cavity, sublingually or in the oral vestibule (between the teeth and lips/cheeks). The user may assist extraction by oral manipulation, such as by chewing and/or sucking or pressing on the outside of the mouth to squeeze the pouch.

There is a need to improve the delivery of the components of the content contained within the smokeless article to improve user perception and experience.

The present disclosure has been devised in light of the above considerations.

### SUMMARY

At its most general, the present disclosure relates to a smokeless article for oral delivery of a nicotinic component.

In a first aspect the present disclosure provides a smokeless article for oral delivery of a nicotinic component, comprising a water-permeable pouch having two or more compartments including a first compartment and a second compartment each containing a content, each compartment (e.g. the content in each compartment) comprising at least one of a nicotinic component and a flavouring, wherein the format of the content contained in the first compartment is different to the format of the content contained in the second compartment.

As used herein, a "smokeless article for oral delivery of a nicotinic component" is an article that does not need to be heated or burned in order to function. The smokeless article is for oral consumption, as used herein, the term "oral consumption" is intended to refer to any oral administration route achieved by placing the smokeless article into the oral cavity. This includes, but is not limited to, buccal, sublingual, periodontal, gingival and ingestion.

Without wishing to be bound by theory, it is thought that the provision of a first and second compartment allows the environment in each compartment to be tuned to improve the performance of the smokeless article. For example, providing two compartments enclosing contents which each have different formats allows, for example, components of each of the compartments to be delivered to a user at relatively different rates. In this way, the profile of delivery of these components, for example of nicotinic components and/or flavourings, to the user can be adjusted in a way which improves user experience, for example by providing a sustained and consistent delivery of those components.

The smokeless article comprises a water-permeable pouch having first and second compartments which each contain contents (i.e., first and second contents respectively). In some examples the compartments are sealed, and in this way completely enclose their respective contents. In some examples the compartments each define an internal volume in which the first and second contents reside. In some examples the compartments enclose their respective contents such that the contents cannot leave the compartments (nor leave the pouch and smokeless article), when the smokeless article is not in use (i.e., in storage), or when it is in use and, for example, scatter in the oral cavity.

In some examples, each of the first and second content independently occupies substantially all of the internal volume of the first and second compartments, respectively. Each of the first and second contents may occupy 80%, 85%, 90%, 95% or 100% of the internal volume of the first and second compartment, respectively.

In some examples, the smokeless article comprises a water-permeable layer which defines the exterior walls of the first and second compartments. Additionally there is provided a partition (optionally also a water-permeable layer) between the first and second compartments to prevent inter-compartment transfer of the contents of one compartment to another compartment (e.g., from the first compartment to the second compartment or vice versa). In use, the water-permeable layer allows water from saliva to enter the smokeless article, resulting in extracting of one or more components from the first and second contents within the smokeless article.

In some examples, the water-permeable layer is porous. In some examples, at least 50% of the pores have a diameter of 50 µm to 200 µm, such as 100 µm to 175 µm or 125 µm or 150 µm. In some examples, at least 50% of the pores have a diameter of at least 100 µm. For example, in some examples at least 55%, 60%, 65%, 70%, 75%, 80%, 85%, 90%, 95% or 100% of the pores have such diameters.

Methods of measuring pore diameter will be known to the skilled person and include, for example, using optical microscopy techniques. Pore diameter here may refer to a mean average pore diameter based on the average pore diameter selected from a sample of pores in the water-permeable layer. The skilled person will be aware of a suitable sample size required to obtain a reliable mean average, which may, for example, 50, 100 or 200 randomly chosen pores.

In some examples the smokeless article may be formed from one or more materials. In some examples, the water permeable layer may be formed from one or more of fibre, paper, cloth and fabric. In some examples, the water-permeable layer may be formed from one or more polymeric materials. In some examples, the polymeric material may be selected from one or more of hydroxypropyl cellulose (HPC), hydroxypropyl methylcellulose (HPMC), polyvinyl alcohol (PVOH), polyvinylpyrrolidone (PVP), polyethylene oxide (PEO) hydroxyethyl cellulose (HEC), polyethylene glycol (PEG), pullulan, sodium alginate, xanthan gum, tragancanth gum, guar gum, acacia gum, arabic gum, polyacrylic acid, maltodextrin, methylmethacrylate copolymer, carboxyvinyl copolymers, starch and gelatin.

The contents of the first and second compartments have different formats to each other. "Format" is used here to refer to the form of the contents, for example the physical form of the contents.

Without wishing to be bound by theory, it is thought that the format in which the contents are provided can affect the rate at which components (for example, nicotinic components, flavourings and flavour modifiers) are released from the contents and so may also affect the rate of delivery of those components to a user when the smokeless article is in use. It is important that smokeless articles provide sustained delivery of components, for example nicotinic components, whereby the components are delivered to a user at a consistent rate for a prolonged period of time.

To provide sustained delivery of a nicotinic component, it is desirable for the smokeless article to provide a first compartment which releases at least one of a nicotinic component and a flavouring relatively quickly (e.g. relatively quickly compared to the release from the second compartment) and a second compartment which releases at least one of a nicotinic component and a flavouring relatively slowly (e.g. relatively slowly compared to the release from the first compartment). This may be achieved by, for example, the different content formats in the first and second compartments providing differing release rates of the at least one of a nicotinic component and a flavouring. This may alternatively be achieved by providing the at least one of a nicotinic component and a flavouring impregnated into or disposed upon the partition between the first and second compartment. In this way, the smokeless article delivers a nicotinic component and/or a flavouring to a user over a prolonged period of time while preferably maintaining a consistent rate of delivery from start of use to end of use.

In some examples, the formats of the contents of the first compartment and the second compartment are each independently at least one selected from the group consisting of a powder, a semi-solid, a monolithic solid and a porous substrate. Semi-solids include, for example, gels, such as gels composed of a cross-linked polymer network. Gels include, for example, hydrogels, including synthetic and natural hydrogels. In some examples, the gel is a synthetic hydrogel, for example a hydrogel comprising one or more of polyvinyl alcohol (PVA), polyethylene glycol (PEG), polyacrylate, and polyvinylpyrrolidone (PVP). In some examples, the gel is a natural hydrogel, for example a hydrogel comprising one or more of gelatine, agar-agar, starch, alginate, agarose (i.e., purified agar-agar or sea-kelp), pectin, and chitosan. In some examples, the hydrogel comprises a polymer which exhibits an upper critical solution temperature (UCST). UCST polymers include, for example, polybetaines, including poly(carboxybetaines), poly(sulfobetaines), and poly(phosphobetaines). In some examples, the hydrogel comprises one or more polybetaines chosen from poly(2-methacryloyloxyethyl phosphorylcholine) (PMPC), poly(sulfobetaine methacrylate) (PSBMA), and poly(carboxybetaine methacrylate) (PCBMA). A "hydrogel" is a three-dimensional network of porous, permeable solid which contains a proportion (e.g., 10% or more) by weight of an interstitial fluid consisting of or comprising water. Porous substrates include, for example, porous pads and sheets in which components are infused. In some examples, the porous substrate has a thickness of 0.1 mm or more, 0.5 mm or more, or 1 mm or more. In some examples, the porous substrate is blotting paper or filter paper. In some examples, the porous substrate is a non-woven fleece membrane or fabric. In some examples, the porous substrate is a wadding material, for example a natural or synthetic wadding material. In some examples, the porous substrate is a synthetic wadding material such as a wadding material comprising polyester. In some examples, the porous substrate is a natural wadding material such as a wadding material comprising one or more of bamboo, cotton, or wool. The contents of the first and second compartments may each independently be a combination of two or more formats. For example, the contents of the first and second compartments may each comprise a combination of two or more of a powder, a semi-solid and a porous substrate. In some examples, one compartment (e.g., the first compartment) comprises a content provided in only one format and another compartment (e.g., the second compartment) comprises a content provided in a combination of formats.

"Natural" (e.g., in terms of "natural hydrogels" or "natural wadding material") refers to materials obtained directly from natural sources. "Synthetic" (e.g., in terms of "synthetic hydrogels" or "synthetic wadding material") refers to materials which have been derived using man-made processes, for example by chemical modification of petroleum.

The contents of the first and second compartments may have any format so long as they are not identical to each other. In some examples, the formats of the contents contained in the first and second compartments are configured to deliver the nicotinic component of the content of the first compartment to a user relatively more quickly than the nicotinic component of the content of the second compartment is delivered to the user.

The contents may, for example, have the same general format type so long as these formats are not identical. For example, the formats of the contents of the first and second compartments may both be porous pads, so long as the format of the porous pads are not identical (e.g. the porous pads may be made of different material which, in the present context, may constitute a different format), such as in a way whereby the porous pads are configured to deliver the nicotinic component of the content of the first compartment to a user relatively more quickly than the nicotinic component of the content of the second compartment. In some examples, the contents may have different format types or combinations of format types.

In some examples, the format of the content of one of the first and second compartment is a gel or a porous substrate and the format of the content of the other of the first and second compartment is a powder or a porous substrate. In some examples, the format of the content of one of the first and second compartment is a gel and the format of the content of the other of the first and second compartment is a powder or a porous substrate. In some examples, the format of the content of one of the first and second compartment is a gel and the format of the content of the other of the first and second compartment is a powder.

In some examples, the format of the content of the first compartment is a gel or a porous substrate and the format of the content of the second compartment is a powder or a porous substrate. In some examples, the format of the content of the first compartment is a gel and the format of the content of the second compartment is a powder or a porous substrate. In some examples, the format of the content of the first compartment is a gel and the format of the content of the second compartment is a powder.

In some examples, at least one of the contents of the first compartment and the second compartment contain a flavour modifier. For example, the contents of the first compartment and the second compartment may both contain flavour modifier.

A flavour modifier is used here to refer to at least one sweetener or component which adjusts the flavour profile (e.g. user perception of flavour) of a flavouring component. The flavour modifier may have the effect of masking or altering (completely or partially) the flavour of the nicotinic component and/or the flavouring of the content(s) perceived by the user. It may be desirable to mask or alter the flavour of nicotinic components, for example, which may be perceived by some users as having a bitter taste.

In some examples, the formats of the contents contained in the first and second compartments are configured to deliver the flavour modifier of the content of the first compartment to a user relatively more quickly than the flavour modifier of the content of the second compartment. Providing the contents in this way allows the smokeless article to provide a sustained delivery of flavour modifier to a user.

In some examples, the format of the content of the first compartment is configured to deliver the flavour modifier and the at least one of a nicotinic component and a flavouring to a user at substantially the same first rate, and the format of the content of the second compartment is configured to deliver the flavour modifier and the at least of a nicotinic component and a flavouring to a user at substantially the same second rate. In this way, contents provide an appropriate release of flavour modifier to mask the flavour of the nicotinic component and/or the flavouring across the entire useful lifetime of the smokeless article. Said first and second rates are typically different from each other. In some cases the first rate is faster than the second rate.

In some examples, one or more of the components of the first compartment (e.g. components of the contents of the first compartment) are identical to one or more corresponding component(s) of the second compartment (e.g. components of the contents of the second compartment). In some examples, one or more of the general components of the first and second compartment (e.g. components of the contents of the first and second compartment) are identical to each other (e.g., the first and second compartment may both comprise a sweetener, such as in their contents). In some examples, one or more of the specific components of the first and second compartment (e.g. components of the contents of the first and second compartment) are identical to each other (e.g., the first and second compartment may both comprise xylitol, such as in their contents).

In some examples, one or more of the components of the first and second compartment (e.g. components of the contents of the first and second compartment) are present (e.g. in their respective contents) in their in substantially or exactly identical relative weight ratios (i.e., the amount of nicotine of the first compartment (e.g. of the content of the first compartment) may be substantially or exactly identical to the amount of nicotine of the second compartment (e.g. of the content of the second compartment)).

Without wishing to be bound by theory, it is thought that providing an article whereby one or more components of the first compartment are identical to one or more components of the second compartment enables the smokeless article to provide a consistent user perception and/or experience across the usable lifetime of the article. The smokeless article comprises a barrier separating the first and second compartments. The barrier prevents physical passage of the content of the first compartment to the second compartment and physical passage of the content of the second compartment to the first compartment. In this way, the formats of the contents in the first and second compartments remain separated from each other.

In some examples, the barrier comprises a weld between two or more surfaces of the water-permeable pouch (e.g. the water-permeable layer defining the outer surface of a smokeless article).

In some examples, the weld is a heat weld, where two or more surfaces of the water-permeable layer are fused together. In some examples heat welding is carried out using heated anvils.

In some examples, the barrier comprises a gel plug. In some examples, the gel plug comprises at least one of a hydrogel or gelatine. In some examples, the gel plug comprises or consists of agar.

In some examples, the barrier comprises a layer of material, for example a water-permeable material as described herein. In some examples, the barrier is water permeable. In some examples, the barrier is not water permeable.

In some examples, depending on the shape of the smokeless article and the first and second compartments, the barrier might be formed from more than one barrier portion, for example more than one weld, or more than one layer of material.

In some examples, the barrier defines an edge or a face of a compartment. In some examples, the first and second compartment are side-by-side in the smokeless article. In such examples it may be that the barrier defines an edge to the compartment. In some examples, the first and second compartment are on top of one another. In such examples it may be that the barrier defines a face of the compartment. In some examples, one compartment (i.e., the first compartment or the second compartment) is contained within the other compartment. In some examples, the containment of the compartment within the other compartment is complete, for example such that the barrier defines all walls of the compartment that is contained. In some examples, the containment is partial, for example such that the barrier does not define all walls of the compartment that is contained.

In some examples, the barrier is disposed along a dimension of the smokeless article such that it defines two substantially or exactly symmetrical compartments and/or two compartments having substantially or exactly identical internal volumes. For example, the barrier may be disposed substantially or exactly midway along a dimension of the smokeless article.

In some examples, the barrier is disposed along a dimension of the smokeless article such that it defines two asymmetrical compartments and/or two compartments which have non-identical internal volumes. For example, the barrier may be disposed along a dimension of the smokeless article while being biased towards one end or side of the smokeless article.

In some examples, the at least one nicotinic compound or flavouring present in the first and/or second compartment is provided by the barrier. For example, the nicotinic compound or flavouring is absorbed into, impregnated into, or provided on the surface or the barrier component. The barrier structure itself may moderate the release of the nicotinic compound or flavouring. In such cases, reference to the first or second compartment comprising at least on of a nicotinic compound or flavouring may mean that the nicotinic compound or flavouring is provided by the barrier, as opposed to the relevant first or second content of the first or second compartment.

In some examples each of the first and second compartments includes a portion of the compartment wall which defines the outer wall of the smokeless article. In this case, neither first nor second compartment is completely formed withing the other with no wall that is part of the outer surface of the smokeless article. In this way, saliva from a user can penetrate both first and second compartments directly, without passing through the other compartment.

In some examples, the colours of the first and second compartment are different. In some examples, the smokeless article and/or the first and second compartments may be coloured or include markings, such as brand logos and text, to improve user perception. The smokeless article may be partially or completely coloured by a colourant.

Colourants may be provided to modify the user impression of the smokeless article. Colourants include whitening agents. Colourants may be selected from one or more of common colourants such as curcumin (E100), turmeric (E100(ii)), riboflavin (E101), riboflavin-5'-phosphate (E101(ii)), tartrazine (E102), quinoline yellow (E104), riboflavin-5-sodium phosphate (E106), yellow 2G (E107), sunset yellow FCF (E110), carmine, cochineal (E120), azorubine (E122), amaranth (E123), ponceau 4R (E124), erythrosine (E127), red 2G (E128), allura red AC (E129), patent blue V (E131), indigotine (E132), brilliant blue FCF (E133) chlorophylls (E140), copper complexes of chlorophyll (E141), green S (E142), caramel (E150a-d), brilliant black BN (E151), carbon (E153), brown FK (E154), brown HT (E155), alfa-, beta- and gamma- carotene (E160a), annatto, bixin, norbixin (E160b), bell pepper (Paprika) extract (E160c), lycopene (E160d), beta- apo-8'-carotenal (E160e), ethyl ester of beta-apo-8'-carotenic acid (E160f), flavoxanthin (E161a), lutein (E161b), cryptoxanthin (E161c), rubixanthin (E161d), violaxanthin (E161e), rhodoxanthin (E161f), canthaxanthin (E1619), citranaxanthin (E161h), beetroot extract (E162), anthocyanins (E163), calcium carbonate (E170), titanium dioxide (E171), iron oxides (E172), aluminium (E173), silver (E174), gold (E175), lithol rubine BK (E180), tannins (E181). The amount of colourant may be up to about 3% by weight of the smokeless article, such as about 0.5% to about 2.5% or about 1% to about 2%.

In some examples, the first and second compartments (e.g. the contents of the first and second compartments) both comprise nicotinic components.

The nicotinic component of the first and second compartments may each individually comprise nicotine freebase, a nicotine salt(s), a nicotine complex(es), and a nicotine solvate(s), or mixtures thereof. In some examples, nicotine salt is selected from the group consisting of nicotine hydrochloride, nicotine dihydrochloride, nicotine monotartrate, nicotine ditartrate, nicotine ditartrate dihydrate, nicotine sulfate, nicotine zinc chloride monohydrate, nicotine salicylate and mixtures thereof. In some examples, the nicotinic component of the first compartment (e.g. of the content of the first compartment) is the same as the nicotinic component of the second compartment (e.g. of the content of the second compartment).

In some examples, the nicotine component is nicotine freebase.

In some examples, the first and second compartments (e.g. the contents of the first and second compartments) may each individually comprise the nicotinic component in an amount of from 0.3 wt.% to 2 wt.%, from 0.4 wt.% to 2 wt.%, from 0.5 wt.% to 2 wt.%, from 0.5 wt.% to 1.9 wt.%, from 0.8 wt.% to 1.8 wt.%, from 1 wt.% to 1.6 wt.%, from 1.2 wt.% to 1.6 wt.%, or from 1.3 wt.% to 1.5 wt.%, based on the total weight, e.g. of the content.

In some examples, the total content of nicotinic component per smokeless article is from 4 to 15 mg. In some examples, the content of nicotinic component per smokeless article is about 7-10 mg. In some examples the content of nicotinic component per smokeless article is about 8 mg.

In some examples, the amount of water in the smokeless article (i.e. the total amount in all present contents) is from 30 wt.% to 60 wt.%, from 30 wt.% to 55 wt.%, from 35 wt.% to 60 wt.%, from 35 wt.% to 55 wt.%, from 30 wt.% to 50 wt.%, or from 30 wt.% to 45 wt.% based on the total weight of content.

In some examples, the smokeless article may have an active lifetime of about 20 minutes to about 60 minutes after being placed in the mouth. In some examples, the smokeless article has an active lifetime of about 25 minutes to 50 minutes after being placed in the mouth. In some examples, the smokeless article has an active lifetime of about 30 minutes to about 45 minutes after being placed in the mouth. As used herein, the term "active lifetime" is intended to refer to the amount of time after being placed in the mouth that the smokeless article provides the user with a perceptible taste and/or physiological experience. For example, the smokeless articles described herein comprises a nicotinic component - the active lifetime may be defined as the in use period of time within which 90 wt% of the available nicotine is released. In other words, the active lifetime may be the duration of time from insertion into the oral cavity for 90 wt% of the total amount of nicotine that is capable of being released during normal use to dissolve into the user's saliva and/or enter the user's bloodstream. It will therefore be appreciated that the active lifetime of a product may vary from user to user and for a user based on oral conditions, in particular extent of salivation. Nonetheless, the skilled person is able to mimic oral conditions to determine the active lifetime in one instance, which can be used as a comparison or analysis point.

In some examples, at least one of the contents of the compartments comprising the nicotinic component comprises tobacco. In some examples, the nicotinic component of either or both of the first compartment and the second compartment is tobacco. "Tobacco" as used herein refers to tobacco, or any tobacco derived product excluding nicotinic components.

In some examples, the contents of at least one of the first and second compartments comprises less than 5 wt% tobacco. In some examples, the contents of at least one of the first and second compartments is free of tobacco. For example, the smokeless article (including both the first and second compartments) may be entirely free of tobacco. In this way, the user may experience a similar or enhanced recreational/pharmaceutical effect as compared to conventional tobacco-containing products without experiencing undesirable components inherent to tobacco (e.g. tobacco flavour).

In some examples, the contents of the first and second compartments may each independently contain a pH buffer. The pH buffer in the content of the first compartment may be the same or different to the pH buffer in the content of the second compartment.

In some examples, the pH buffer is at least partially responsible for setting, maintaining or altering the pH of the first and/or second contents.

In some examples, the pH buffer is at least partially responsible for setting, maintaining or altering the pH of the contents of the first and/or second compartments.

As used herein, the terms "pH regulator", "buffer", "pH stabiliser", "pH modifier", and "pH adjuster" interchangeably refer to components of the first and/or second content used to control pH. Within these definitions, the terms "pH regulator", "buffer" and "pH stabiliser" refer to compounds that have the capacity to resist pH change to some level so providing some ability to maintain pH in a specific buffer range as compared to just altering the pH.

Examples of suitable pH buffers include ammonia, ammonium carbonate, ammonium chloride, sodium carbonate, calcium lactate, and calcium carbonate. These components may have a buffering action, which helps to maintain the pH at the desired level.

In some examples, the content of the first compartment comprises a nicotinic component and a pH buffer. In some examples, the content of the second compartment comprises a nicotinic component and a pH buffer. In some examples, the contents of the first and second compartment both comprise a nicotinic component and a pH buffer.

In some examples, the contents of the first and second compartment both comprise a nicotinic component and a pH buffer, wherein the type of nicotinic component and/or pH buffer in the content of the first compartment is identical to the type of nicotinic component and/or pH buffer in the content of the second compartment (e.g., the contents of the first and second compartment may both comprise nicotine freebase and sodium carbonate).

The term "carbonate" may be used to refer to bicarbonate, carbonate, and mixtures thereof. In some examples, "carbonate" is carbonate. In some examples "carbonate" is bicarbonate. In some examples, "carbonate" is a mixture of carbonate and bicarbonate.

In some examples, the first and second compartments (e.g. the contents of the first and second compartments) comprise a flavouring. In this way, the smokeless article may provide a sustained and consistent release of flavouring to a user. In some examples, smokeless articles having first and second compartments which each comprise flavourings may further comprise nicotinic component(s) in the first compartment, the second compartment, or both the first and second compartment such as in the contents of the relevant compartment. In some examples, the smokeless articles in which both the first and second compartments comprise flavourings (e.g. in their contents) are free of nicotinic components.

Flavouring may be provided in solid or liquid form. Suitable flavourings include coffee, eucalyptus, menthol, liquorice, peppermint, spearmint, chocolate, fruit flavour (including e.g. citrus, cherry etc.), vanilla, spice (e.g. ginger, cinnamon) and tobacco flavour. The flavouring may be evenly dispersed throughout the content in which it is contained or may be provided in isolated locations and/or varying concentrations throughout the content in which it is contained. The flavouring may be provided in, or on, the barrier between the first and second compartments. As used herein, the term "flavouring" denotes a compound having a desirable taste, aroma or both. In some examples, the flavouring is vanilla (i.e. comprises vanillin).

In some examples, the contents of the first and second compartments may each independently comprise one or more flavourings (e.g. peppermint flavour) in an amount of from above 0 wt.% to 10 wt.%, from 1 wt.% to 10 wt.%, from 1 wt.% to 8 wt.%, from 1 wt.% to 6 wt.%, from 2 wt.% to 8 wt.%, from 2 wt.% to 7 wt.%, or from 3 wt.% to 7 wt.% based on the total weight of each content.

In some examples, the contents of the first and second compartments may each independently comprise a sweetener as a flavour modifier. In some examples, the content of the first compartment comprises a sweetener. In some examples, the content of the second compartment comprises a sweetener. In some examples, the contents of both the first and second compartments comprises a sweetener.

Sweeteners may be provided to modify the user taste perception and, in particular, overcome bitter flavours that result from other substances. Suitable sweeteners include honey, sugar, brown sugar, glucose, fructose, sucrose, aspartame, xylitol, maltitol, saccharin sodium, glycyrrhizin tripotassium liquorice, jujube, acesulfame K, sucralose or a mixture thereof. In some cases the sweetener may be xylitol.

In some examples, the contents of the first and second compartments may each independently comprise one or more sweeteners in an amount of from 0 wt.% to 5 wt.%, from 0.01 wt.% to 4 wt.%, from 0.01 wt.% to 3 wt.%, from 0.5 wt.% to 3 wt.%, or from 1 wt.% to 3 wt.% based on the total weight of each content.

In some examples, the contents of the first and second compartments each independently comprise flavour modifiers comprising a sweetener.

In some embodiments, the first compartment has a content comprising a flavour modifier comprising a sweetener.

In some embodiments, the second compartment has a content comprising a flavour modifier comprising a sweetener.

In some examples, the contents of the first and second compartments may each independently comprise a salt. In some examples, the content of the first compartment comprises a salt. In some examples, the content of the second compartment comprises a salt.

In some examples of the smokeless article, the salts may be provided to maintain the osmolarity of the content within the smokeless article. Salts contribute to the overall positive user experience of the smokeless article, such as pleasant mouth feel. Examples of salts which may be used include sodium chloride, potassium chloride, magnesium chloride and calcium chloride.

In some examples, the contents of the first and second compartments may each independently comprise a humectant. In some examples, the content of the first compartment comprises a humectant. In some examples, the content of the second compartment comprises a humectant.

Humectants may be provided to control moisture content thereby preventing the smokeless article from drying out during storage and reducing the amount of saliva wetting required before the user experience begins. Suitable humectants include polyhydric alcohols (e.g. propylene glycol (PG), triethylene glycol, 1 ,2-butane diol and glycerol such as vegetable glycerine (VG)) and their esters (e.g. glycerol mono-, di- or tri-acetate). The humectant component may consist of a single humectant compound or may consist of two or more different humectant compounds.

In some examples, the contents of the first and second compartments may each independently comprise an active ingredient. In some examples, the content of the first compartment comprises an active ingredient. In some examples, the content of the second compartment comprises an active ingredient. In some examples, the active ingredient is a biologically and/or pharmaceutically active ingredient.

Active ingredients (i.e. biologically/pharmacologically active compounds) are provided to produce a pharmacological effect in the user. Suitable active compounds include the group consisting of: nicotine, cannabinoids, caffeine, opiates and opioids, cathine and cathinone, kavalactones, mysticin, beta-carboline alkaloids, salvinorin A, together with any combinations, functional equivalents to, and/or synthetic alternatives of the foregoing. Active ingredients (i.e. biologically/pharmacologically active compounds) may also have additive properties. In some examples, active ingredients are present at 1-3 wt% relative to the total weight of the content of the compartment.

In some examples, the contents of the first and second compartments may each independently comprise a filler. In some examples, the content of the first compartment comprises a filler. In some examples, the content of the second compartment comprises a filler.

In some examples, the filler comprises one or more natural fibres. In some examples the natural fibres comprise one or more of micro-crystalline cellulose, wheat fibre, bamboo fibre, apple fibre, citrus fibre, sugar cane fibre, rice fibre, psyllium fibre, potato fibre, pea fibre, and oat fibre.

Fillers may be provided to increase the volume of the smokeless article and the volume of the first and/or second compartments (e.g., by increasing the volume contained within the smokeless article and to strengthen the content(s) of the first and/or second compartments).

In general, suitable fillers include calcium carbonate, calcium phosphate, corn starch, grains, lactose, polysaccharides (e.g. maltodextrin), polyols, sugars (e.g. dextrose, mannitol, xylitol, sorbitol), natural fibres (e.g. non-tobacco fibres), microcrystalline cellulose, cellulose and cellulose derivatives (e.g. finely divided cellulose), lignocellulose fibres (e.g. wood fibres), jute fibres and combinations thereof. In some cases the filler comprises wheat fibres. In some cases the filler comprises bamboo fibres. The use of a filler improves the overall mouth-feel of the smokeless article.

In some examples the filler comprises micro-crystalline cellulose and one of wheat fibres or bamboo fibres.

In some examples, the filler content is about 30 to 70 wt% of the content of the compartment. In some examples, the filler content is about 40 to 60 wt% of the content of the compartment.

In some examples, the contents of the first and second compartments each independently comprise plant material. In some examples, the content of the first compartment comprises plant material. In some examples, the content of the second compartment comprises plant material. In some examples, the plant material functions as a filler.

Plant material may be provided for physical integrity and may function as a natural source of substances such as, for example, biologically/pharmacologically active compounds, flavourings, pH stabilisers etc. The plant material may comprise at least one plant material selected from the list including *Amaranthus dubius, Arctostaphylos uva-ursi* (Bearberry), *Argemone mexicana, Amica, Artemisia vulgaris,* Yellow Tees, *Galea zacatechichi, Canavalia maritima* (Baybean), *Cecropia mexicana* (Guamura), *Cestrum noctumum, Cynoglossum virginianum* (wild comfrey), *Cytisus scoparius, Damiana, Entada rheedii, Eschscholzia califomica* (California Poppy), *Fittonia albivenis, Hippobroma longiflora, Humulus japonica* (Japanese Hops), *Humulus lupulus* (Hops), *Lactuca virosa* (Lettuce Opium), *Laggera alata, Leonotis leonurus, Leonurus cardiaca* (Motherwort), *Leonurus sibiricus* (Honeyweed), *Lobelia cardinalis, Lobelia inflata* (Indian-tobacco), *Lobelia siphilitica, Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum, Salvia dorrii* (Tobacco Sage), Salvia species (Sage), *Scutellaria galericulata, Scutellaria lateriflora, Scutellaria nana, Scutellaria* species (Skullcap), *Sida acuta* (Wireweed), *Sida rhombifolia, Silene capensis, Syzygium aromaticum* (Clove), *Tagetes lucida* (Mexican Tarragon), *Tarchonanthus camphoratus, Tumera diffusa* (Damiana), *Verbascum* (Mullein), *Zamia latifolia* (Maconha Brava) together with any combinations, functional equivalents to, and/or synthetic alternatives of the foregoing.

In some examples, the filler may have a particle size of 10-500 µm, 10-450 µm, 10-400 µm, 10-350 µm, 10-300 µm, 10-250 µm, 10-200 µm, 10-150 µm, 10-100 µm, 10-50 µm, 15-50 µm, 10-450 µm, 15-45 µm, 20-50 µm, 20-45 µm, 20-40 µm, or 25-35 µm.

In some examples, the filler may have a particle size of 15-500 µm, 20-500 µm, 50-500 µm, 100-500 µm, 150-500 µm, 200-500 µm, 250-500 µm, 250-450 µm, 250-400 µm, 250-350 µm, 260-340 µm, 270-330 µm, 280-320 µm, 290-310 µm, or 295-305 µm.

As used herein, the term "particle size" when referring to fibres may indicate the maximum size of the longest dimension of the fibres. For example, a particle size of 50 µm indicates that in the population of fibres, the maximum fibre length is 50 µm. Alternatively, the term "particle size" when referring to fibres may indicate the average size of the longest dimension of the fibres.

Particles having the desired particle size may be obtained by passing a population of fibres through a sieve of corresponding mesh size. For example, to obtain fibres of particle size 300 µm (i.e. a maximum fibre length of 300 µm), a population of fibres are passed through a sieve with 300 µm diameter apertures in the mesh. In this way, fibres with a length of 300 µm or less pass through the mesh and fibres longer than 300 µm are retained by the mesh. The fibres which pass through the mesh may then be used in the smokeless article of the invention, having a particle size of 300 µm. Fibres of a desired particle size are also available from commercial suppliers such as Jelu-werk.

In some examples, the contents of the first and second compartments may each independently comprise a binder. In some examples, the content of the first compartment comprises a binder. In some examples, the content of the second compartment comprises a binder.

Suitable binders include starches and/or cellulosic binders such as methyl cellulose, ethyl cellulose, hydroxypropyl cellulose, hydroxyethyl cellulose and carboxymethyl cellulose, gums such as xanthan, guar, arabic and/or locust bean gum, organic acids and their salts such as alginic acid (sodium alginate), agar and pectins.

In some examples, the contents of the first and second compartments may each independently comprise a stabiliser. In some examples, the content of the first compartment comprises a stabiliser. In some examples, the content of the second compartment comprises a stabiliser.

Stabilisers are provided to prevent decomposition or degradation over time during storage by, for example, retarding oxidation or unwanted biological activity. Stabilisers may be selected from the group consisting of antioxidants including vitamin E, such as tocopherol, ascorbic acid, sodium pyrosulfite, butylhydroxytoluene, butylated hydroxyanisole, edetic acid and salts thereof; and preservatives including citric acid, tartaric acid, lactic acid, malic acid, acetic acid, benzoic acid, sorbic acid and salts thereof.

In some examples, the contents of the first and second compartments may each independently have a shelf-life (for example, measured as no noticeable change in user perception and/or safety) of at least 4 months, at least 5 months, at least 6 months, at least 7 months, or at least 8 months.

In some examples, the smokeless article may have a mass of about 0.1 g to 5.0 g. In some examples the smokeless article has a mass of about 0.5 g to about 4.0 g. In some examples, the smokeless article has a mass of about 1.0 g to about 3.0 g.

In some examples, the smokeless article has a length of about 30 to 40 mm, about 32 to 38 mm, or about 35 mm. In some examples, the smokeless article has a width of about 10 to 20 mm, about 12 to 16 mm, or about 14 mm. In some examples, the smokeless article has a seal length of about 1 to 10 mm, about 2 to 6 mm, or about 4 mm. In some examples, the smokeless article has a seal area (width x seal length) of about 10 to 200 mm², about 24 to 96 mm², or about 56 mm². In some examples, the smokeless article has a depth of about 5 mm, about 4 mm, or about 3 mm. Smokeless articles of these dimensions may be comfortable for a user when the smokeless article is placed next to the gum.

Seal length and seal area are used here to refer to the portions of the smokeless article which are used to form a seal between the elements which make up the smokeless article and do not have an internal volume which define compartments.

The smokeless article may have any shape. In some examples, the smokeless article is rectangular with angular corners and/or rounded corners. Rounded corners may improve user comfort. Alternatively, the smokeless article may have another shape, for example a triangular, square, oval, circular, or any other imaginable shape.

The compartments may have any shape. In some examples, the compartments have the same shape as each other. Alternatively, the compartments may have different shapes to each other. In some examples, the compartments have the same dimensions and/or internal area as each other. Alternatively, one or more compartments may be larger or smaller than the remaining compartment(s). In some examples, the compartments have the same shapes as each other, for example rectangular or square shapes. Alternatively, the compartments may have triangular, oval, circular or any other imaginable shape. In some examples, one or more compartments may have a different shape to the remaining compartment(s).

In some examples, the smokeless article comprises a third and optionally further compartment(s). In some examples, when the smokeless article comprises a third and optionally further compartment(s), the smokeless article comprises more than one barrier, and each barrier may comprise more than one barrier portion (i.e. more than one weld or layer of material).

In some examples, there are 1, 2, 3 or 4 barriers. Where there are multiple barriers, the barrier may be formed in the same, or a different way (for example, one barrier may be a weld, the other a separate layer of material).

The third and further compartments may be defined as the first and second compartments have been defined above. The contents of the third and further compartments may be defined as the contents of the first and second compartments have been defined above.

In some examples, the formats of the contents of at least one of the third and further compartments are different to the formats of either of the contents of the first and second compartments. For example, a smokeless article comprising first, second and third compartments may have first, second and third contents enclosed respectively therein which each have different formats.

In some examples, the formats of the contents of at least one of the third and further compartments are identical to the format of one of the contents of either the first or second compartment. For example, a smokeless article comprising first, second and third compartments may have first and third contents having identical formats enclosed in the first and third compartments, while the second compartment encloses a second content having a different format to the first and third contents.

In some examples, at least one of the third and further compartments do not comprise content(s) having nicotinic component(s) or flavouring(s). For example, a smokeless article comprising first, second and third compartments may have first and second contents having at least one of a nicotinic component and a flavouring enclosed in the first and second compartments and a third content which does not have any nicotinic components or flavourings enclosed in the third compartment.

In some examples, at least one of the third and further compartments do not comprise content(s). For example, a smokeless article comprising first, second and third compartments may have first and second contents enclosed in the first and second compartments and a third compartment which does not enclose any content.

In a second aspect, the present disclosure provides a method of manufacturing the smokeless article described herein, comprising: cutting a water-permeable membrane to form shaped elements for forming the smokeless article; and thermoforming to seal the shaped elements to form the smokeless article.

In some examples, the step of cutting may involve laser cutting techniques.

In some examples, the method of manufacturing the smokeless article comprises thermoforming to partially seal the shaped elements. In such examples, filling of the first and second compartments takes place to fill the partially formed compartments. In some examples, the content(s) of the first and/or the second compartment(s) are present before thermoforming and sealing is complete.

In a third aspect, the present disclosure provides a kit comprising a plurality of smokeless articles according to the first aspect. In some examples, the kit comprises a container. In some examples, the kit comprises 2 to 20 smokeless articles.

In a fourth aspect, the present disclosure provides a use of a smokeless article as described herein to deliver a sustained release of at least one of a nicotinic component and a flavouring to a user. In some examples, the use comprises delivering a sustained release of a flavour modifier to mask or alter the flavour of the at least one of a nicotinic component and a flavouring.

The preceding summary is provided for purposes of summarizing some examples to provide a basic understanding of aspects of the subject matter described herein. Accordingly, the above-described features should not be construed to narrow the scope or spirit of the subject matter described herein in any way. Moreover, the above and/or proceeding examples may be combined in any suitable combination to provide further examples, except where such a combination is clearly impermissible or expressly avoided. Other features, aspects, and advantages of the subject matter described herein will become apparent from the following text and the accompanying drawings.

### BRIEF DESCRIPTION OF THE FIGURES

Aspects, features and advantages of the present disclosure will become apparent from the following description of examples in reference to the appended drawings in which like numerals denote like elements.

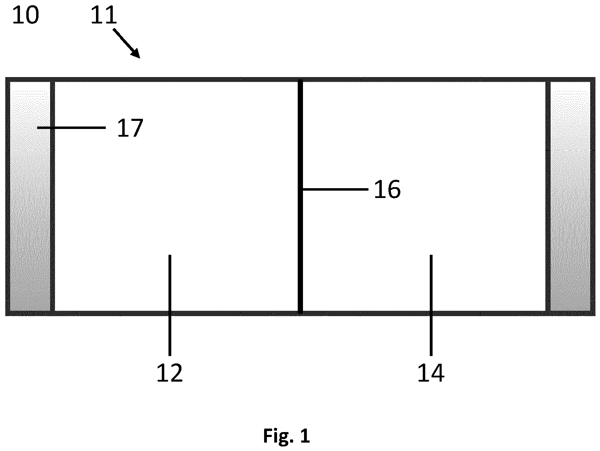

**Fig. 1** shows a cross sectional view of an embodiment of a smokeless article according to the invention.

### DETAILED DESCRIPTION OF EMBODIMENTS

Before describing several examples implementing the present disclosure, it is to be understood that the present disclosure is not limited by specific construction details or process steps set forth in the following description and accompanying drawings. Rather, it will be apparent to those skilled in the art having the benefit of the present disclosure that the systems, apparatuses and/or methods described herein could be embodied differently and/or be practiced or carried out in various alternative ways.

Unless otherwise defined herein, scientific and technical terms used in connection with the presently disclosed inventive concept(s) shall have the meanings that are commonly understood by those of ordinary skill in the art, and known techniques and procedures may be performed according to conventional methods well known in the art and as described in various general and more specific references that may be cited and discussed in the present specification.

Any patents, published patent applications, and non-patent publications mentioned in the specification are hereby incorporated by reference in their entirety.

All examples implementing the present disclosure can be made and executed without undue experimentation in light of the present disclosure. While particular examples have been described, it will be apparent to those of skill in the art that variations may be applied to the systems, apparatus, and/or methods and in the steps or in the sequence of steps of the methods described herein without departing from the concept, spirit, and scope of the inventive concept(s). All such similar substitutions and modifications apparent to those skilled in the art are deemed to be within the spirit, scope, and concept of the inventive concept(s) as defined by the appended claims.

The use of the term "a" or "an" in the claims and/or the specification may mean "one," as well as "one or more," "at least one," and "one or more than one." As such, the terms "a," "an," and "the," as well as all singular terms, include plural referents unless the context clearly indicates otherwise. Likewise, plural terms shall include the singular unless otherwise required by context.

The use of the term "or" in the present disclosure (including the claims) is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used in this specification and claim(s), the words "comprising, "having," "including," or "containing" (and any forms thereof, such as "comprise" and "comprises," "have" and "has," "includes" and "include," or "contains" and "contain," respectively) are inclusive or open-ended and do not exclude additional, unrecited elements or method steps.

Unless otherwise explicitly stated as incompatible, or the physics or otherwise of the embodiments, examples, or claims prevent such a combination, the features of examples disclosed herein, and of the claims, may be integrated together in any suitable arrangement, especially ones where there is a beneficial effect in doing so. This is not limited to only any specified benefit, and instead may arise from an "ex post facto" benefit. This is to say that the combination of features is not limited by the described forms, particularly the form (e.g. numbering) of example(s), embodiment(s), or dependency of claim(s). Moreover, this also applies to the phrase "in one embodiment," "according to an embodiment," and the like, which are merely a stylistic form of wording and are not to be construed as limiting the following features to a separate embodiment to all other instances of the same or similar wording. This is to say, a reference to 'an,' 'one,' or 'some' embodiment(s) may be a reference to any one or more, and/or all embodiments, or combination(s) thereof, disclosed. Also, similarly, the reference to "the" embodiment may not be limited to the immediately preceding embodiment. Further, all references to one or more embodiments or examples are to be construed as non-limiting to the claims.

Fig.1 shows a schematic of an embodiment of the smokeless article 10 of the present invention. The smokeless article 10 comprises a water-permeable pouch 11 having two compartments 12, 14. The first compartment 12 is substantially filled with a first content (not shown) and the second compartment 14 is substantially filled with a second content (not shown). The two compartments 12, 14 are separated by a barrier 16, such that the first content and the second content cannot transfer between the first compartment 12 and the second compartment 14.

The smokeless article 10 is rectangular with angular corners at the edges of a seal area 17 which seals the shaped elements which form the smokeless article 10. The smokeless article 10 has a length of 35 mm, a width of 14 mm, a seal length of 4 mm and a seal area of 56 mm². The compartments 12, 14 are approximately the same size and have square shapes.

In the embodiment of Fig. 1, the first content in the first compartment 12 and the second content in the second compartment 14 both contain at least one of a nicotinic component and a flavouring.

### EXAMPLES

### Preparation of contents

A powder content was prepared according to the following method at the lab scale using a food processor.
1. All materials were dispensed into appropriately sized beakers using a 2 decimal placed balance to determine weights.
2. Filler, pH buffer, salt, sweetener, and humectant are added to the food processor and mixed at full speed for 5 minutes.
3. Whilst mixing, the nicotine in water and flavouring is added over the course of 2-3 minutes.
4. The blend is mixed for a further 5 minutes, resulting in a final powdery content.
   The smokeless article is then prepared by the following subsequent steps, making use of the powdery content described above in a first compartment and a content having a different format (i.e., a non-powdery content, for example a semi-solid, a monolithic solid or a porous substrate content) in the second compartment).
5. A pouch comprising two compartments partitioned barrier formed by a heat weld between two surfaces of the water-permeable layer of the pouch is provided. The powder content is filled into a first compartment, which is open or partially unformed.
6. Prior, simultaneously, or subsequently to the first compartment of the pouch being filled with the powder content, the second content is filled into the second compartment, which is open or partially unformed.
7. The open or partially unformed first and second compartments are sealed and sprayed with the water "water (sprayed)".

By way of example, a suitable final powder content includes one based on the composition table set out below.

| **Material** | **%w/w (weight/weight)** |
|---|---|
| Nicotine | 1.4 - 1.42 |
| Water | 7.25 - 17.3 |
| Flavouring | 4.03 - 6.94 |
| Filler | 36.99 - 49.11 |
| Humectant | 2 - 11.5 |
| Salt | 1.93 - 2 |
| Sweetener | 2.68 - 4.08 |
| pH Buffer | 0.05 - 2.67 |
| Water (sprayed) | 17.5 - 30.92 |

## Claims

1. A smokeless article for oral delivery of a nicotinic component, comprising a water-permeable pouch having two or more compartments including a first compartment and a second compartment each containing a content, each compartment, or each content, comprising at least one of a nicotinic component and a flavouring, wherein the format of the content contained in the first compartment is different to the format of the content contained in the second compartment.

2. The smokeless article according to claim 1, wherein the formats of the contents contained in the first and second compartments are configured to deliver the at least one of a nicotinic component and a flavouring of the content of the first compartment to a user relatively more quickly than the at least one of a nicotinic component and a flavouring of the content of the second compartment.

3. The smokeless article according to claim 1 or 2, wherein the contents of the first compartment and the second compartment both comprise a nicotinic component.

4. The smokeless article according to claim any one of claims 1 to 3, wherein at least one of the contents of the first compartment and the second compartment contain a flavour modifier.

5. The smokeless article according to claim 4, wherein the contents of the first compartment and the second compartment both comprise a flavour modifier.

6. The smokeless article according to claim 5, wherein the formats of the contents contained in the first and second compartments are configured to deliver the flavour modifier of the content of the first compartment to a user relatively more quickly than the flavour modifier of the content of the second compartment.

7. The smokeless article according to claim 5 or 6, wherein the format of the content of the first compartment is configured to deliver the flavour modifier and the at least one of a nicotinic component and a flavouring to a user at substantially the same first rate, and the format of the content of the second compartment is configured to deliver the flavour modifier and the least one of a nicotinic component and a flavouring to a user at substantially the same second rate.

8. The smokeless article according to any one of claims 1 to 7, wherein the format of the contents of the first compartment and the second compartment is at least one selected from the group consisting of a powder, a semi-solid such as a gel, a monolithic solid, and a porous substrate.

9. The smokeless article according to claim 8, wherein the format of the content of one of the first and second compartment is a gel or a porous substrate and the format of the content of the other of the first and second compartment is a powder or a porous substrate.

10. The smokeless article according to claim 9, wherein the format of the content of one of the first and second compartment is a gel and the format of the content of the other of the first and second compartment is a powder.

11. The smokeless article according to any one of claims 1 to 10, wherein the first and second compartments are separated by a barrier comprising a weld between two or more surfaces of the water-permeable pouch.

12. A method of manufacturing the smokeless article of any one of claims 1 to 11, comprising: cutting a water-permeable membrane to form shaped elements for forming the smokeless article; and thermoforming to seal the shaped elements to form the smokeless article.

13. A kit comprising a plurality of smokeless articles according to any one of claims 1 to 11.

14. Use of a smokeless article according to any one of claims 1 to 11 to deliver a sustained release of at least one of a nicotinic component and a flavouring to a user.

15. The use according to claim 13, comprising to deliver a sustained release of a flavour modifier to mask or alter the flavour of the at least one of a nicotinic component and a flavouring.
